# EUROPEAN PATENT SPECIFICATION

(11) **EP 0 997 527 B1**
(45) Date of publication and mention of the grant of the patent: **09.06.2010**
(21) Application number: 99307443.4
(22) Date of filing: 21.09.1999
(51) Int. Cl.: C12N 9/96, C12N 9/08, C07K 14/805, C07K 14/80

(54) **Stabilisation of peroxidase conjugates**
Stabilisierung von Peroxidase-Konjugaten
Stabilisation de conjugués de peroxidases

(30) Priority: 22.09.1998 GB 9820641; 01.09.1999 GB 9920670
(43) Date of publication of application: 03.05.2000
(73) Proprietor: Ortho-Clinical Diagnostics, Amersham, Buckinghamshire HP7 OHJ (GB)
(72) Inventor: Yearwood, Graham de Lisle, London N11 2TX (GB); Kilmartin, Patrick, Didcot, Oxfordshire OX11 7RT (GB); Mann, Verouka Zsuzsanna, London WC1N 2NW (GB); Dawkes, Adrian Charles, Windsor, Berkshire SL4 3PF (GB); Hourd, Paul, Harrow, London HA1 4JN (GB); Edwards, John, Little Kingshill, Buckinghamshire HP16 0EZ (GB)
(74) Representative: Mercer, Christopher Paul

(56) References cited:
- WO-A-92/19707
- WO-A-93/16195
- WO-A1-95/02046
- US-A- 5 512 448
- NAGASAKI T ET AL: "Novel chemical modification of myoglobin by an alcohol-responsive phenylboronic acid function" CHEM LETT,October 1994 (1994-10), pages 1945-1948, XP002129187
- LIU X ET AL: "Studies on oriented and reversible immobilization of glycoprotein using novel boronate affinity gel" J MOL RECOGN, vol. 9, no. 5-6, September 1996 (1996-09), pages 462-467, XP002129188
- NAGASAKI T. ET AL: 'Novel chemical modification of myoglobin by an alcohol-responsive phenylboronic acid function' CHEMISTRY LETTERS, CHEMICAL SOCIETY OF JAPAN, TOKYO, JP 01 October 1994, pages 1945 - 1948, XP002129187 ISSN: 0366-7022

## Description

### Field of Invention

The present invention relates to a process for stabilising peroxidases conjugated to other biologically active molecules and to a solution comprising and stabilizing a peroxidase conjugate, for use in analytical procedures.

### Background of the Invention

Peroxidases, in particular horseradish peroxidase (HRP), are enzymes that are commonly used in analytical procedures including immunoassays. Many formats are known and used widely for the measurement of antigens, antibodies, haptens, and other analytes. Some examples are competitive and immunometric assays. In competitive immunoassays, a labelled analyte competes with the analyte in a sample for binding to an immobilised analyte binding species. Thus, increasing the concentration of the analyte in the sample reduces the labelled analyte which becomes immobilised. In immunometric assays, a labelled analyte binder complexes with analyte in the sample and an immobilised analyte binder. In this type of assay, increasing the concentration of the analyte in the sample results in an increase in the labelled analyte binder which becomes immobilised. In both types of immunoassay, the immobilised labelled analyte is separated from the unbound labelled analyte, and either the immobilised or unbound HRP activity is measured.

Reagents for use in analytical procedures, such as immunoassays, must remain active after manufacture, shipping, and periods of storage. During these periods they may be subject to relatively high or low temperatures which affect the activity. Therefore, it is a commercial advantage to provide a stable reagent containing the peroxidase-labelled material or other active biological components.

This is not readily achieved for many biological materials. For example, enzyme-labelled molecules and other complex biomolecules are widely used in immunoassays, and it has been observed that the activity of the enzyme or of the active biomolecule can decrease with time, especially at the low concentrations used in immunoassay procedures. Biological activity is found to decrease more rapidly as the storage temperature increases. One way to slow down or prevent a decrease in the activity of an enzyme or enzyme conjugated to an active molecule is to add a stabilising agent to the enzyme in solution in an amount sufficient to achieve stabilisation.

Methods for stabilisation of peroxidases or HRP include the addition of polyethylene glycol and calcium salts (US Patent 4,728,023); the addition of 8-anilino-1-naphthalene sulfonic acid (FRG Patent 3,100,076); the addition of phenol and substituted phenols with one or more substituents from C₁-C₃ lower alkyl, chlorine and bromine(US Patent 4,764,468); the addition of 4-aminoantipyrine (European Patent 070,992); the addition of polyvalent ions of Group III and IV (US Patent 4,169,012) and the addition of a 4-hydroxy or 4-alkoxyarylacetamide (US Patent 5,372,932). Stabilisation of both HRP and other biomolecules has been achieved by the addition of a compound having a benzene ring substituted with at least one hydroxyl group and one group of Hammett sigma value (σₚ) -0.20 ≤ x ≥ +0.24 or an amide (US Patent 5,516,672).

Although the currently known additives improve the stability of peroxidase and other biological molecules, there is still a need for further improvement. In particular, reagent formulations which have improved stability at room temperature and the elevated temperatures experienced during transport of reagents are desired. Some of the known additives can interact with proteins and other components which are frequently used in analytical reagent formulations and in some cases can affect the immunological reactions which are utilized in immunoassays. Additives which stabilise the active components of the formulations and which have no deleterious effects on other components are also desired.

Organoboron compounds in aqueous solutions interact strongly with many molecules. The interaction, which can be by a number of different mechanisms, has been exploited in the purification of various materials. For example, 3-aminophenylboronic acid attached to a solid support is used in the fractionation of glycoproteins. Organoborates can inhibit many enzymes, affect animal metabolism and are recognised bacteriostats (see Boronate Ligands in Biochemical Separations, Amicon Corporation, Danvers, Publication 507, 1981, for a comprehensive review). Organoboron compounds have also been found to increase the light output from chemiluminescent reactions catalysed by HRP (PCT Publication No. WO 93/16195) alone or in combination with a phenolic or aromatic amine enhancer (PCT Publication No. WO 94/23060).

Organoboron compounds also have been used in electron transfer electrodes (PCT Publication No. WO 85/02861).

Aryl boronic acids are a family of compounds within the organoboron group. Aryl boronic acids have been used in formulations of liquid detergents which contain proteolytic enzymes (PCT Publication No. WO 92/19707). At high concentrations of aryl boronic acid, the proteolytic enzymes are prevented from degrading other protein components of the detergent formulation. On subsequent dilution in wash water the proteolytic enzymes become re-active.

The use of boronic acid or borinic acid derivatives as enzyme stabilizers is described in WO 95/02046.

Nagasaki, T. et al. (Chem. Lett. 1994, 1945-1948) indicates that phenylboronic acid-substituted Myoglobin (a peroxidase) is more stable than non-substituted Myoglobin.

Liu, X. et al. (J. Mol. Recogn., vol. 9, No. 5-6, 1996, 462-467 describes that catechol [2-(diethylamino)carbonyl-4-bromomethyl]phenylboronate may be used to immobilize horseradish peroxidase.

Surprisingly, we have found that aryl boronic acid compounds can be added as a stablilising agent to slow down or prevent a decrease in the activity of peroxidase or peroxidase that has been conjugated to another molecule (hereinafter peroxidase labelled conjugate or conjugate), particularly HRP or HRP-conjugate intended for use in an immunoassay.

### Summary of the Invention

Therefore, an object of the present invention is to provide a method as recited in claim 1. Said method notably entails stabilising a peroxidase labelled conjugate by adding at least one aryl boronic acid compound to the conjugate reagent. The stabilised compositions are useful in analytical procedures. They are particularly useful in enzyme immunoassays.

One embodiment of the present invention is a solution as recited in claim 9, which solution has a pH of 7.0 or below 7.0 and in which a peroxidase conjugate is stabilised by at least one aryl boronic acid.

A preferred embodiment is either the composition or method above wherein the peroxidase is horseradish peroxidase.

### DETAILED DESCRIPTION

The present invention relates to the use of aryl boronic acid compounds as stabilisers of HRP. The stabiliser is an aryl boronic acid of the formula ArB(OH)₂ where Ar is phenyl or naphthyl.

The aryl structure can be either unsubstituted or substituted with one or more substituents selected from the group consisting of: alkyl, substituted alkyl, aryl, substituted aryl excluding substitution with hydroxyl, ether, amine, substituted amine, azo, amide, halogen, nitro, thiol, thiol derivative, aldehyde, acid, acid salt, ester, sulfonate, phosphonate, trimethyl silyl. Some examples of substituted and unsubstituted compounds are:

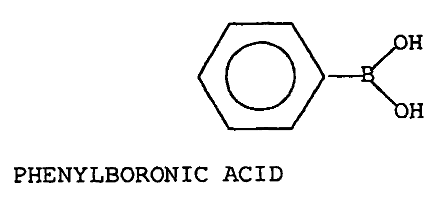

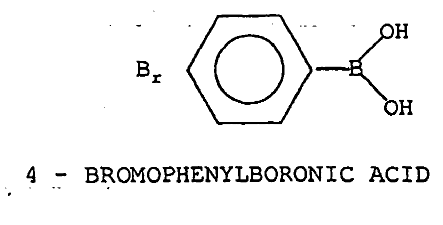

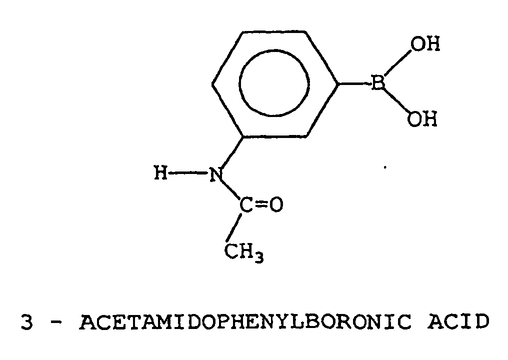

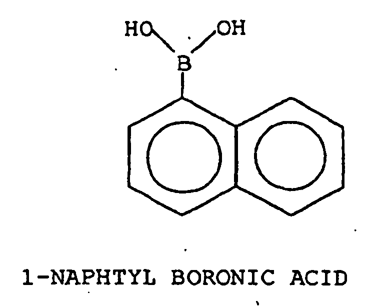

Preferred compounds for use in the present invention are 4-bromophenylboronic acid, 3-acetamidophenylboronic acid, and phenylboronic acid, which are shown above. A preferred embodiment of the present invention is a stabilised HRP composition comprising bromophenylboronic acid or a method of stabilising HRP using bromophenylboronic acid. In particular, 4-bromophenylboronic acid showed desirable solubility and suitable stability effects. However, one skilled in the art will know how to choose an aryl boronic acid compound that is suitable for a particular situation.

The aryl boronic acid stabiliser can be added at any desired point of time to the peroxidase conjugate. The reagent ingredients, the stabiliser itself and the peroxidase conjugate, as well as the various other parts of the assay reagent may be present in dissolved form. The stabiliser should be added in amount sufficient to achieve stabilisation. Preferably, the stabiliser is added in an amount that is 0.01% to 0.1% weight per volume (% w/v).

In one embodiment of the invention, the stabiliser can be added to a liquid reagent which is subsequently dried since a more uniform distribution of the stabiliser and thus a better action in the case of additions close to the lower limit of the effective range is hereby achieved. For example the aryl boronic acid compound can be added to the peroxidase conjugate solution before lyophilisation or after reconstitution of the lyophilisate with an aqueous solvent. In the latter case, the solvent can, of course, first also be mixed with the stabiliser and then added to the lyophilisate for dissolving it. If, for example, the peroxidase conjugate is a component in a dry analytical assay element, e.g., thin-film or slide elements, the stabiliser would preferably be added to a coating solution prior to the coating process. It is however, contemplated that the stabiliser could be added after the coating process is completed. For example, by spraying it on.

The peroxidase is conjugated to another molecule that is biologically active or immunologically effective. Such a molecule is referred to herein as an "active molecule". An active molecule is one which can be used in an analytical procedure. Said active molecule would be an antibody or a fragment thereof, an antigen or a hapten, protein, glycoprotein, peptide, protein mimetic, nucleic acid sequence(s), steroid, hormone or derivatives of any of the foregoing or a compound made of more than one or a combination of the foregoing.

When practicing the present invention, it may be desirable to add also a preserving agent. Common preserving agents include, for example, 2-hydroxpyridine-N-oxide, chloroacetamide, N,N-methylene-bis - (N-1-hydroxymethyl)-2,5-dioxo-4-imidazolidinyl)-urea, 5 bromo-5-nitro-1,3-dioxan.

In solutions which have a pH close to or below 7.0, boronates are uncharged and hydrophobic interactions and charge transfer interactions cause tight binding to many molecules, particularly proteins. Surprisingly we have found that this interaction gives a stabilising effect on biologically active molecules.

Several assays were performed wherein aryl boronic acid was added to the reagents containing a peroxidase label. Assays can be carried out using an enzyme label which can be attached to the ligand to form a labelled ligand or to the ligand binder to form a labelled ligand binder. Enzymes such as peroxidases, e.g. horseradish peroxidase (HRP), are preferred labels. According to the invention the enzyme is a peroxidase. There is a variety of types of this enzyme. Horseradish peroxidase is the most preferred type and it is readily available.

It is within the skill of the ordinary artisan to determine a suitable substrate for a given enzyme label. The substrate can be a material which is directly acted upon by the enzyme label or a material that is involved in a series of reactions which involve enzymatic reaction of the label.

The effectiveness and advantages of the invention are further illustrated by the following examples.

### Examples

### Example 1: Stabilisation of an HRP-antibody conjugate

A monoclonal antibody (raised against human follicle stimulating hormone) was conjugated to HRP using succinimidyl-4- (N-maleimidomethyl) cyclohexane-1-carboxylate (SMCC) and N-succinimidyl S-acetylthioacetate (SATA) obtained from Pierce and Warriner, Chester, U.K. The HRP-antibody conjugate was then biotinylated using BIOTIN-XX-NHS (Calbiochem, Beeston, U.K.) The biotinylated HRP-antibody was desalted on a PD-10 column (Pharmacia Biotech, St.Albans, U.K.) following the manufacturer's recommended protocol using 100mM potassium phosphate buffer pH 6.0 containing 300mM sodium chloride as an eluting buffer. The conjugate was quantified by UV adsorption, diluted in test reagents with various additives and then stored at 2-8 degrees C or 37 degrees C. The activity of the conjugate in the reagents was then tested by incubating 100 microlitres of the reagent in a VITROS ECi streptavidin coated microwell (Ortho-Clinical Diagnostics, U.K.) for 15 minutes at 37 degrees C. During the incubation the biotinylated HRP-antibody conjugate becomes immobilised on the surface of the microwell by the reaction of the biotin with streptavidin. After washing the microwells, the activity of the immobilised HRP-antibody complex was measured using an enhanced luminescence reaction (US Patent 5,372,931). VITROS ECi Signal Reagent, containing luminogenic substrates (a luminol derivative and a peracid salt) and an electron transfer agent , was added to the microwells. The HRP in the bound conjugate catalyzes the oxidation of the luminol derivative, producing light. The electron transfer agent (a substituted acetanilide) increases the level of light produced and prolongs its emission. The light signals from the microwells were measured using an AMERLITE Analyzer (Ortho-Clinical Diagnostics, U.K.).

The biotinylated HRP-antibody conjugate described above was diluted in 0.1 M potassium phosphate buffer pH 7.0 to give a concentration of 10 ng/mL. Magnesium-depleted KATHON (Rohm and Haas, U.K.) was added as a preservative (1% w/v) and bovine serum albumin (1% w/v) was added to prevent non-specific adsorption of the conjugate to the surface of the microwells. Each aryl boronic acid stabilising agent was formulated as a 200 mg/mL solution in dimethylformamide (DMF) and added to the conjugate solution to give the desired molarity of stabiliser. The conjugate solutions were stored at 2-8 degrees C or 37 degrees C for 2 hours, 24 hours, 96 hours and 9 weeks. The activity of the HRP in the conjugate was then measured as described above. The results are shown in Table 1.

**Table 1 - Activity of HRP after storage of an HRP - antibody conjugate.**

| Additive | HRP activity after 2 hours storage (%) | HRP activity after 24 hours storage (%) | HRP activity after 96 hours storage (%) | HRP activity after 9 weeks storage (%) |
|---|---|---|---|---|
| Control - no additive, Stored at 2-8 degrees C | 100 | 105.2 | 96.8 | 81.8 |
| No additive, Stored at 37 degrees C | 97.7 | 41.8 | 6.6 | - |
| 4-bromophenyl-boronic acid (0.1% w/v), stored at 37 degrees C | 104.3 | 113.4 | 100.0 | 60.1 |
| 3-acetamino-phenylboronic acid (0.1% w/v), stored at 37 degrees C | 100.5 | 106.5 | 97.1 | Insufficient sample |
| 4-phenylboronic acid (0.1% w/v), stored at 37 degrees C | 100.3 | 91.1 | 94.3 | 51.1 |
| DMF (0.5% v/v), stored at 37 degrees C | 98.2 | 64.4 | 8.5 | - |

HRP activity is the light output expressed as a percentage of the control light output (no additive - 2 hours storage at 2-8 degrees C).

It can be seen from Table 1 that the activity of HRP decreased markedly after storage at 37 degrees C in the absence of a stabiliser. HRP activity is retained during storage at 2-8 degrees C or in the presence of the aryl boronic acid compounds. Even after 9 weeks storage at 37 degrees C, the HRP activity is still over 50% of the starting activity in the presence of a stabiliser.

### Example 2: Stabilisation of an HRP-testosterone conjugate

HRP was chemically coupled to testosterone-3-carboxymethyloxime by reaction with dicyclohexylcarbodiimide and N-hydroxysuccinimide (Aldrich Chemical Co., Gillingham, U.K.). The purified conjugate was diluted to a concentration of 60 ng/mL in a 50mM phosphate buffer pH 7.0 containing 1.3% sodium chloride, 2% sucrose, 0.5% magnesium depleted KATHON, 0.2% bovine serum albumin and 600 microgram/L DANAZOL as a steroid releasing agent. The conjugate solution was stored at 25 degrees C in the presence of various concentrations of 4-bromophenylboronic acid and the HRP conjugate activity was then measured using a competitive immunoassay procedure.

A monoclonal antibody to testosterone was biotinylated using NHS-LC-biotin (Pierce and Warriner, Chester, U.K.) and diluted to 10 mg/L in a 50mM potassium phosphate buffer pH 7.3 containing 1.3% sodium chloride, 70 mM EDTA, 0.5% magnesium depleted KATHON, 1% human plasma, 0.05% normal mouse serum and 600 microgram/L of DANAZOL. Charcoal stripped human plasma (25 microlitres) was incubated with 50 microlitres of the HRP-testosterone conjugate solution and 100 microlitres of the biotinylated antibody solution in a VITROS ECI streptavidin coated microwell (Ortho-Clinical Diagnostics, U.K.) for 30 minutes at 37 degrees C. During the incubation, the HRP-testosterone conjugate becomes bound to the biotinylated antibody and the complex is immobilised on the surface of the streptavidin coated microwell through the reaction of biotin with streptavidin. After washing the microwell, the HRP activity was measured using VITROS ECi Signal Reagent in a VITROS ECi Analyzer. The results are shown in Table 2.

**Table 2 -Stabilisation of an HRP-testosterone conjugate**

| Concentration of 4-bromophenyl boronic acid (% w/v) | HRP activity at 0 days storage (%) | HRP activity at 7/8 days storage (%) | HRP activity at 21 days storage (%) | HRP activity at 26/28 days storage (%) |
|---|---|---|---|---|
| 0.0 | 100 | 89.3 | 83.4 | 77.1 |
| 0.01 | 100 | 96.3 | 88.0 | - |
| 0.02 | 100 | 92.8 | - | 86.4 |
| 0.03 | 100 | 94.7 | - | 89.0 |
| 0.04 | 100 | 98.1 | - | 91.0 |
| 0.05 | 100 | 95.3 | 92.2 | 92.1 |
| 0.1 | 100 | 98.7 | 99.9 | - |

HRP activity is the light output expressed as a percentage of the 0 day storage light output.

In the absence of a stabiliser, the activity of the HRP-testosterone conjugate had decreased to 83.4% after 21 days storage at 25 degrees C. With increasing amounts of bromophenylboronic acid the conjugate was more stable. After 26-28 days storage in the absence of a stabiliser, the activity of the HRP-testosterone conjugate had decreased to 77.1%. Again with increasing amounts of bromophenylboronic acid the conjugate was more stable.

### Example 3: Stabilisation of an HRP-antibody/antigen complex

HRP was chemically coupled to a monoclonal antibody to hepatitis B core antigen (HBc) using succinimidyl-4-(N-maleimidomethyl) cyclohexane-1-carboxylate (SMCC) and N-succinimidyl S-acetylthioacetate (SATA). The purified HRP conjugate was diluted to a concentration of 0.057 microgram/mL in a 20mM sodium phosphate buffer pH 6.2 containing 2.0% sodium chloride, 2.0% magnesium-depleted KATHON, 2.0% bovine serum albumin and 0.065 microgram/mL recombinant HBc (Chiron Corporation, Emeryville, California, U.S.A.). The HRP-labelled anti-HBc antibody combines with the HBc antigen to form an HRP-labelled antibody/antigen complex. The solution was stored at 25 degrees C or 37 degrees C in the presence or absence of 4-bromophenylboronic acid and the HRP antibody/antigen complex activity was then measured using an immunoassay procedure.

A monoclonal antibody to human IgM was biotinylated using BIOTIN-X-NHS (Calbiochem, Beeston, U.K.) and diluted to 0.75 mg/L in a 25mM potassium phosphate/15mM sodium tetraborate buffer pH 8.2 containing 0.5% bovine serum albumin and 0.96% magnesium-depleted KATHON. Human plasma containing IgM antibodies to HBc was diluted 1 in 20 in a 100mM potassium phosphate buffer containing 0.9% sodium chloride, 5% bovine serum albumin and 1% BRONIDOX (Henkel K.G., Germany). 25 microlitres of the diluted plasma was incubated with 145 microlitres of the biotinylated anti-IgM antibody solution in a VITROS ECi streptavidin coated microwell (Ortho-Clinical Diagnostics, U.K.) for 15 minutes at 37 degrees C. During the incubation, any IgM antibodies in the plasma become immobilised on the surface of the streptavidin coated microwell by reaction with the biotinylated anti-IgM and the reaction of biotin with streptavidin. After washing the microwell, 100 microlitres of the HRP-labelled antibody/antigen complex solution is added to the microwell and incubated for a further 15 minutes at 37 degrees C. During this reaction, the HBc in the complex reacts with any antibodies to HBc immobilised on the microwell. After washing the microwell the activity of HRP bound to the surface of the microwell was measured using VITROS ECi Signal Reagent in a VITROS ECi Analyzer. The results are shown in Table 3.

**Table 3 - Stabilisation of an HRP-antibody/antigen complex**

| Concentration of 4-bromophenyl boronic acid (% w/v) | HRP activity at 0 days storage (%) | HRP activity at 1 week storage at 25 degrees C (%) | HRP activity at 15 weeks storage at 25 degrees C (%) | HRP activity at 1 week storage at 37 degrees C (%) |
|---|---|---|---|---|
| 0.0 | 100 | 1.0 | 0.9 | 0.5 |
| 0.01 | 100 | 38.0 | 0.0 | 0.0 |
| 0.1 | 100 | 61.0 | 45.1 | 15.3 |

HRP activity is the light output expressed as a percentage of the 0 day storage light output.

In the absence of a stabiliser, the activity of the HRP conjugate had decreased to less than 1% after storage for 1 week. With 0.01% bromophenylboronic acid the conjugate retained 38% of its activity after 1 week storage at 25 degrees C, and with 0.1% bromophenylboronic acid 45% of the activity was retained after 15 weeks storage at 25 degrees C and 15% of the activity was retained after 1 week storage at 37 degrees C.

### Example 4: HRP conjugate used in a dry, thin-film format (example provided for information)

In addition to the working examples above, as was discussed previously, the present teaching is contemplated to be useful in a dry immunoassay analytical element. Therefore, the following examples describe subject-matter that should result in an enzyme conjugate having the beneficial properties described herein.

Dry analytical elements comprising HRP for determining the presence or amount of an analyte, such as, but not limited to glucose, triglycerides, lipase, glycerol, cholesterol, cholesterol esters are well known. Dry immunoassay analytical elements comprising HRP-conjugates are also well known, such as immunoassay elements for determining C-Reactive Protein (CRP), digoxin, phenytoin and the like.

The stabiliser may be introduced into the element in any layer or zone including the spreading layer, subbing layers and/or reagent layers using well known methods, for example hopper and gravure coating techniques. In a preferred element HRP and stabiliser are present together.

An analytical element, comprising HRP and an aryl boronic acid stabiliser in the same layer, for the determination of cholesterol would have the following composition and format:

| Layer | Component | Range of Dry Coverage (g/m²) |
|---|---|---|
| Spreading Layer | Barium sulfate | 70-140 |
| | Cellulose Acetate Polyurethane resin | 6-12 0.5-1.5 |
| | | |
| Reagent Layer | Potassium phosphate buffer (pH 5.5 - 6.5) | 1-2 |
| | TRITON X-100 (surfactant) | 5-11 |
| | HRP | 5,000-160,000 International Units (IU) |
| | Aryl boronic acid stabiliser | 0.01 - 10 |
| | | |
| | Cholesterol oxidase | 2,000 IU - 4000 IU |
| | Cholesterol ester hydrolase | 1,500 IU-12,000 IU |
| | 2-(3,5-Dimethyoxy-4-hydroxyphenyl-4,5-bis (4-dimethylaminophenyl) imidazole | 0.8-3 |
| | 5,5-Dimethyl-1,3-cyclohexane dione | 0.2-0.8 |
| | | |
| Subbing Layer | Poly(N-isopropylacrylamide) | 0.2-0.8 |
| | | |
| Binder Layer | Gelatin (hardened) | 10-25 |
| | Potassium phosphate buffer | 0.1-2 |
| | TRITON X-200E surfactant | 0.005 - 0.02 |
| | | |
| Support | Poly(ethylene terephthalate) | |

The element can be mounted into slides, kept in strips or other suitable form and stored under conditions to be tested. The reflectance density measured using a relectance spectrometer obtained with cholesterol standards using a control element (for example, a fresh element) can be compared with elements stored under test conditions. The location of the stabiliser in the element and the amount of the stabiliser can be readily altered until the desired stability is obtained, as would be well known to the skilled artisan.

### Example 5: Dry Immunoassay Element for the detection of Phenytoin (example provided for information)

An immunoassay analytical element, comprising a phenytoin-HRP conjugate and aryl boronic acid stabiliser in the same layer, for the determination of phenytoin has the following composition and format:

| Layer | Component | Dry Coverage (g/m²) |
|---|---|---|
| Gravure | Phenytoin-HRP conjugate | 1.0 X 10⁻⁶ - 100 X 10⁻⁶ |
| | Magenta dye | 0.01 - 0.05 |
| | 3-(N-Morpholino)propanesul fonic acid buffer, pH 7.0 | 0.001-0.01 |
| | Bovine serum albumin | 1.0 X 10-4 -5.0 X 10-4 |
| | Polyacrylamide | 0.05 X 10-3 - 3.0 X 10-3 |
| | Trehalose | 0.1 -0.4 |
| | Aryl boronic acid stabiliser | 0.0001 - 1.0 |
| | | |
| Bead Spreading Layer | Poly(vinyltoluene-co-methylacrylic acid) beads (20- 40 microns) | 100-160 |
| | Poly(methyl acrylate-co-sodium-2-acrylamido-2-methylpropane sulfonate-co-2-acetoacetoxyethyl methacrylate) | 1.8 - 3.5 |
| | Gylcerol | 1.0 - 3.0 |
| | Bovine serum albumin | 0.05 -3.0 |
| | Mannitol | 0.05 - 3.0 |
| | Dimedone | 0.1-1.0 |
| | N-[TRIS (hydroxymethyl)m ethyl]-2- aminoethane sulfonic acid buffer, pH 7.0 | 0.1 - 1.0 |
| | Aryl boronic acid stailiser | 0.01 - 10.0 |
| | | |
| Receptor Layer | Poly(N- | 0.1- 1.0 |
| | isopropylacrylamide-co- methacrylic acid-co-N,N'-methylenebisacrylamid e) | |
| | 4,5-Bis-(4-dimethylaminophenyl) -2-(3,5-dimethoxy-4-hydroxyphenyl) imidazole | 0.1- 2.0 |
| | Dimedone | 0.01-1.0 |
| | TETRONIC T908 surfactant | 0.01-0.04 |
| | OLIN 10G surfactant | 0.005-0.02 |
| | TX 100 surfactant N-[TRIS (hydroxymethyl)m ethyl]-2- aminoethane sulfonic acid buffer, pH 7.0 | 0.01-0.03 0.01-0.02 |
| | Poly[styrene-co-p-(2-chloroethylsulfonyl methyl)styrene] beads having a phenytoin antibody bound thereto | 0.05 - 0.3 |
| | | |
| Gelatin Layer | Gelatin | 5-20 |
| | N-[TRIS (hydroxymethyl)m ethyl]-2- | 2.0-8.0 |
| | aminoethane sulfonic acid buffer, | pH 7.0 |
| | TX-100 surfactant | 0.01-0.03 |
| | Bis (vinylsulfonylmeth yl)ether | 0.05-0.30 |
| | | |
| Support | Poly(ethylene terephthalate) | |

As above, the element can be mounted into slides, kept in strips or other suitable form and stored under conditions to be tested. The reflectance density obtained with phenytoin standards using a control element (for example, a fresh element) can be compared with elements stored under test conditions. The location of the stabiliser in the element and the amount of the stabilser can be readily altered until the desired stability is obtained, as would be well known to the skilled artisan.

The aryl boronic acid compounds will act to stabilise dry analytical elements comprising HRP directed to analytes, in addition to the cholesterol element described above and dry immunoassay analytical elements comprising HRP-conjugates directed to immunassays in addition to that described above for phenytoin.

### Example 6 - Stabilisation of myoglobin

Myoglobin, hemoglobin and cytochrome C are proteins which have peroxidase activity. The enzymic activity of these proteins has been utilized in immunoassays in which they have been detected by their enzyme-catalysed luminescent reaction of luminol with peroxide (GB Patent No. 2,063,469).

Myoglobin has been shown to be a useful marker for acute myocardial infarction (US Patent No. 5,744,358). Standard reference solutions of myoglobin are used in the measurement of this analyte by immunological reactions, and the stability of these solutions can be improved by the addition of an aryl boronic acid compound.

### Immunometric assay of myoglobin

Two mouse monoclonal antibodies to myoglobin were obtained from BiosPacific, USA. HRP was chemically coupled to one of the myoglobin monoclonal antibodies using sulfosuccinimidyl-4-(N-maleimidomethyl) cyclohexane-1-carboxylate (sulfo-SMCC) and N-hydroxysuccinimidyl S-acetylthioacetate (NHS-SATA) obtained from Pierce and Warriner, U.K. The purified HRP conjugate was diluted to a concentration of 3 microgram/mL in a 100mM potassium phosphate buffer pH 6.5 containing 1.0% magnesium-depleted KATHON and 0.5% bovine serum albumin.

The second myoglobin monoclonal antibody was biotinylated using biotin-LC-NHS (Pierce and Warriner, U.K.) and diluted to 3 microgram/mL in a 100mM potassium phosphate buffer pH 6.5 containing 5.0% bovine serum albumin and 1.0% magnesium-depleted KATHON.

Standard solutions of human cardiac myoglobin obtained from BiosPacific, Emeryville, USA were prepared in a 100mM potassium phosphate buffer pH 7.0 containing 1.0% magnesium depleted KATHON, 5.0% bovine serum albumin, 10mM sodium chloride and 1mM EDTA.

10 microlitres of a standard myoglobin solution were incubated with 70 microlitres of the biotinylated monoclonal antibody solution and 70 microlitres of the HRP-labelled monoclonal antibody solution in a VITROS ECi streptavidin coated microwell (Ortho-Clinical Diagnostics, U.K.) for 8 minutes at 37 degrees C. During the incubation, the biotinylated antibody becomes immobilised on the surface of the streptavidin coated microwell by the reaction of biotin with streptavidin. At the same time, myoglobin in the standard solution becomes immobilised to the biotinylated antibody and also binds with the HRP-labelled monoclonal antibody. After washing the microwell the activity of HRP bound to the surface of the microwell was measured using VITROS ECi Signal Reagent in a VITROS ECi Analyzer. The activity of the HRP bound to the microwells, measured as the light output in the Analyzer, is proportional to the immunoreactivity of the myoglobin added in the assay. A response curve of light output against myoglobin immunoreactivity was constructed using a series of standard myoglobin solutions covering the range 0 to 1757 ng/ml.

### Stability of myoglobin

Human cardiac myoglobin was dissolved at a concentration of 350 ng/ml in 100mM potassium phosphate buffer pH 7.0 containing 1.0% magnesium depleted KATHON, 5.0% bovine serum albumin, 10mM sodium chloride and 1mM EDTA. 4-bromophenylboronic acid and 3-acetamidophenylboronic acid were each formulated as a 100 mg/mL solution in ethanol and added to the myoglobin solution to give the desired concentration of stabiliser. The myoglobin solutions were then stored at 25 degrees C or 37 degrees C and after 1 week and 2 weeks the amount of immunoreactive myoglobin was measured using the immunometric assay described above. The effect of 4-bromophenylboronic acid and 3-acetamidophenylboronic acid on myoglobin stability is shown in Table 4, and the effect of varying the concentration of 4-bromophenylboronic acid is shown in Table 5.

**Table 4 - Stabilisation of myoglobin**

| | Myoglobin Immunoreactivity | | | | |
|---|---|---|---|---|---|
| Additive | 0 days storage (%) | 1 week storage at 25 deg C (%) | 2 weeks storage at 25 deg C (%) | 1 week storage at 37 deg C (%) | 2 weeks storage at 37 deg C (%) |
| Control -No additive | 100 | 66 | 49 | 14 | 12 |
| 4-bromophenyl boronic acid (0.12% w/v) | 100 | 79 | 79 | 33 | 25 |
| 3-acetamido phenyl boronic acid (0.12% w/v) | 100 | 84 | 80 | 53 | 42 |

Myoglobin immunoreactivity is expressed as a percentage of the 0 days storage immunoreactivity.

In the absence of a stabiliser, the immunoreactivity of the myoglobin decreased to 49% after storage for 2 weeks at 25 degrees C. With 0.12% 4-bromophenylboronic acid the myoglobin immunoreactivity retained 79% of its activity after 2 weeks storage at 25 deg C, and with 0.12% 3-acetamidophenylboronic acid, 80% of the activity was retained after 2 weeks storage at 25 degrees C.

In the absence of a stabiliser, the immunoreactivity of the myoglobin decreased to 12% after storage for 2 weeks at 37 degrees C. With 0.12% 4-bromophenylboronic acid the myoglobin immunoreactivity retained 25% of its activity after 2 weeks storage at 37 deg C, and with 0.12% 3-acetamidophenylboronic acid, 42% of the activity was retained after 2 weeks storage at 37 degrees C.

**Table 5 - Stabilisation of myoglobin**

| | Myoglobin Immunoreactivity | | | | |
|---|---|---|---|---|---|
| Concentration of 4-bromo phenylboronic acid (% w/v) | 0 days storage (%) | 1 week storage at 25 deg C (%) | 2 weeks storage at 25 deg C (%) | 1 week storage at 37 deg C (%) | 2 weeks storage at 37 deg C (%) |
| 0 | 100 | 66 | 49 | 14 | 12 |
| 0.1 | 100 | 68 | 46 | 22 | 39 |
| 0.2 | 100 | 68 | 57 | 22 | 26 |
| 0.3 | 100 | 74 | 65 | 28 | 25 |
| 0.4 | 100 | 73 | 66 | 37 | 32 |
| 0.5 | 100 | 77 | 74 | 26 | 26 |

Myoglobin immunoreactivity is expressed as a percentage of the 0 days storage immunoreactivity.

In the absence of a stabiliser, the immunoreactivity of the myoglobin had decreased to 49% after storage for 2 weeks at 25 degrees C and to 12% after 2 weeks at 37 degrees C. With increasing amounts of 4-bromophenylboronic acid the myoglobin was more stable. In the presence of 0.5 % 4-bromophenylboronic acid, 74% of the myoglobin immunoreactivity was retained after 2 weeks at 25 degrees C and 26% was retained after 2 weeks at 37 degrees C.

It should be noted that the present invention includes all modifications falling within the scope of the following claims.

## Claims

1. A method for stabilising in a solution which has a pH of 7.0 or below 7.0 an immunologically active compound having peroxidase activity and conjugated to an active molecule selected from the group consisting of: an antibody or a fragment thereof, an antigen, a hapten, a protein, a glycoprotein, a peptide, a protein mimetic, a nucleic acid molecule, a steroid, a hormone, or a derivative of any of the foregoing, said method comprising: adding an amount effective for stabilising said conjugated compound having peroxidase activity of at least one aryl boronic acid compound of formula ArB(OH)₂ wherein Ar is phenyl or naphthyl that is either unsubstituted or is substituted with one or more substituents selected from the group consisting of alkyl, substituted alkyl, aryl, substituted aryl, ether, amine, substituted amine, azo, amide, halogen, nitro, thiol, thiol derivative, aldehyde, acid, acid salt, ester, sulfonate, phosphonate, and trimethyl silyl, wherein when the aryl boronic acid compound is substituted with a substituted aryl, the substituent in the substituted aryl is not hydroxyl.

2. The method of claim 1, wherein the aryl boronic acid compound is 4-bromophenylboronic acid.

3. The method of claim 1, wherein the aryl boronic acid compound is 3-acetamidophenylboronic acid.

4. The method of any one of claims 1 to 3, wherein the immunologically active compound is myoglobin.

5. The method of any one of claims 1 to 3, wherein the immunologically active compound is a peroxidase.

6. The method of claim 5, wherein the peroxidase is horseradish peroxidase.

7. The method of any one of claims 1 to 6, wherein the solution is an aqueous solution.

8. The method of claim 7, wherein the amount of aryl boronic acid compound added is from 0.01 percent to 0.5 percent, preferably about 0.1 percent, by weight per volume.

9. A solution which has a ph of 7.0 or below 7.0, which solution comprises an immunologically active compound having peroxidase activity and conjugated to an active molecule selected from the group consisting of an antibody or a fragment thereof, an antigen, a hapten, a protein, a glycoprotein, a peptide, a protein mimetic, a nucleic acid molecule, a steroid, a hormone, or a derivative of any of the foregoing, said solution additionally comprising at least one aryl boronic acid compound of formula ArB(OH)₂ wherein Ar is phenyl or naphthyl that is either unsubstituted or is substituted with one or more substituents selected from a group consisting of alkyl, substituted alkyl, aryl, substituted aryl, ether, amine, substituted amine, azo, amide, halogen, nitro, thiol, thiol derivative, aldehyde, acid, acid salt, ester, sulfonate, phosphonate, and trimethyl silyl, wherein when the aryl boronic acid compound is substituted with a substituted aryl, the substituent in the substituted aryl is not hydroxyl.

10. The solution of claim 9, wherein the aryl boronic acid compound is 4-bromophenylboronic acid.

11. The solution of claim 9, wherein the aryl boronic acid compound is 3-acetamidophenylboronic acid.

12. The solution of any one of claims 9 to 11, wherein the reagent further comprises a preserving agent.

13. The solution of any one of claims 9 or 12, wherein the immunologically active compound is myoglobin.

14. The solution of any one of claims 9 to 12, wherein the immunologically active compound is a peroxidase.

15. The solution of claim 14, wherein the peroxidase is horseradish peroxidase.

16. The solution of any one of claims 9 to 15, wherein the solution is an aqueous solution.

17. The solution of claim 16, wherein the aryl boronic acid compound is present in an amount that is from 0.01 percent to 0.5 percent, preferably about 0.1 percent, by weight per volume.

## Patentansprüche

1. Verfahren zur Stabilisierung einer immunologisch aktiven Verbindung, die Peroxidase-Aktivität aufweist und an ein aktives Molekül konjugiert ist, das ausgewählt ist aus der Gruppe, bestehend aus: einem Antikörper oder einem Fragment davon, einem Antigen, einem Hapten, einem Protein, einem Glykoprotein, einem Peptid, einem Proteinmimetikum, einem Nukleinsäuremolekül, einem Steroid, einem Hormon oder einem Derivat von einem der Vorstehenden, in einer Lösung, die einen pH von 7,0 oder unter 7,0 aufweist, wobei das Verfahren umfasst: Zugeben einer zur Stabilisierung der konjugierten Verbindung mit Peroxidase-Aktivität wirksamen Menge wenigstens einer Arylboronsäureverbindung von Formel ArB(OH)₂, worin Ar Phenyl oder Naphthyl ist, das entweder unsubstituiert ist oder mit einem oder mehreren Substituenten substituiert ist, die ausgewählt sind aus der Gruppe, bestehend aus Alkyl, substituiertem Alkyl, Aryl, substituiertem Aryl, Ether, Amin, substituiertem Amin, Azo, Amid, Halogen, Nitro, Thiol, Thiol-Derivat, Aldehyd, Säure, Säuresalz, Ester, Sulfonat, Phosphonat und Trimethylsilyl, wobei, wenn die Arylboronsäureverbindung mit einem substituierten Aryl substituiert ist, der Substituent im substituierten Aryl nicht Hydroxyl ist.

2. Verfahren nach Anspruch 1, wobei die Arylboronsäureverbindung 4-Bromphenylboronsäure ist.

3. Verfahren nach Anspruch 1, wobei Arylboronsäureverbindung 3-Acetamidophenylboronsäure ist.

4. Verfahren nach einem der Ansprüche 1 bis 3, wobei die immunologisch aktive Verbindung Myoglobin ist.

5. Verfahren nach einem der Ansprüche 1 bis 3, wobei die immunologische aktive Verbindung eine Peroxidase ist.

6. Verfahren nach Anspruch 5, wobei die Peroxidase Meerrettich-Peroxidase ist.

7. Verfahren nach einem der Ansprüche 1 bis 6, wobei die Lösung eine wässrige Lösung ist.

8. Verfahren nach Anspruch 7, wobei die zugegebene Menge Arylboronsäureverbindung von 0,01 Prozent bis 0,5 Prozent, vorzugsweise etwa 0,1 Prozent, bezogen auf Gewicht pro Volumen, beträgt.

9. Lösung, die einen pH von 7,0 oder unter 7,0 aufweist, wobei diese Lösung eine immunologisch aktive Verbindung umfasst, die Peroxidase-Aktivität aufweist und an ein aktives Molekül konjugiert ist, das ausgewählt ist aus der Gruppe, bestehend aus einem Antikörper oder einem Fragment davon, einem Antigen, einem Hapten, einem Protein, einem Glykoprotein, einem Peptid, einem Proteinmimetikum, einem Nukleinsäuremolekül, einem Steroid, einem Hormon oder einem Derivat von einem der Vorstehenden, wobei die Lösung zusätzlich wenigstens eine Arylboronsäureverbindung von Formel ArB(OH)₂ umfasst, worin Ar Phenyl oder Naphthyl ist, das entweder unsubstituiert ist oder mit einem oder mehreren Substituenten substituiert ist, die ausgewählt sind aus der Gruppe, bestehend aus Alkyl, substituiertem Alkyl, Aryl, substituiertem Aryl, Ether, Amin, substituiertem Amin, Azo, Amid, Halogen, Nitro, Thiol, Thiol-Derivat, Aldehyd, Säure, Säuresalz, Ester, Sulfonat, Phosphonat und Trimethylsilyl, wobei, wenn die Arylboronsäureverbindung mit einem substituierten Aryl substituiert ist, der Substituent im substituierten Aryl nicht Hydroxyl ist.

10. Lösung nach Anspruch 9, wobei die Arylboronsäureverbindung 4-Bromphenylboronsäure ist.

11. Lösung nach Anspruch 9, wobei Arylboronsäureverbindung 3-Acetamidophenylboronsäure ist.

12. Lösung nach einem der Ansprüche 9 bis 11, wobei das Reagens weiter ein Konservierungsmittel umfasst.

13. Lösung nach einem der Ansprüche 9 bis 12, wobei die immunologisch aktive Verbindung Myoglobin ist.

14. Lösung nach einem der Ansprüche 9 bis 12, wobei die immunologische aktive Verbindung eine Peroxidase ist.

15. Lösung nach Anspruch 14, wobei die Peroxidase Meerrettich-Peroxidase ist.

16. Lösung nach einem der Ansprüche 9 bis 15, wobei die Lösung eine wässrige Lösung ist.

17. Lösung nach Anspruch 16, wobei die Arylboronsäureverbindung in einer Menge vorliegt, die von 0,01 Prozent bis 0,5 Prozent, vorzugsweise etwa 0,1 Prozent, bezogen auf Gewicht pro Volumen, beträgt.

## Revendications

1. Procédé pour stabiliser dans une solution ayant un pH de 7,0 ou inférieur à 7,0 un composé immunologiquement actif possédant une activité peroxydase et conjugué à une molécule active choisie dans le groupe constitué par : un anticorps ou fragment de celui-ci, un antigène, un haptène, une protéine, une glycoprotéine, un peptide, une protéine mimétique, une molécule d'acide nucléique, un stéroïde, une hormone, ou un dérivé de l'une quelconque des molécules précédentes, ledit procédé comprenant : l'ajout d'une quantité efficace pour stabiliser ledit composé conjugué possédant une activité peroxydase d'au moins un composé d'acide arylboronique de formule ArB(OH)₂, Ar étant un phényle ou un naphtyle qui est soit non substitué, soit substitué par un ou plusieurs substituants choisis dans le groupe constitué par un alkyle, un alkyle substitué, un aryle, un aryle substitué, un éther, une amine, une amine substituée, un composé azoïque, un amide, un halogène, un nitro, un thiol, un dérivé de thiol, un aldéhyde, un acide, un sel d'acide, un ester, un sulfonate, un phosphonate, et un triméthylsilyle, dans lequel, quand le composé d'acide arylboronique est substitué par un aryle substitué, le substituant dans l'aryle substitué n'est pas un hydroxyle.

2. Procédé selon la revendication 1, dans lequel le composé d'acide arylboronique est l'acide 4-bromo-phénylboronique.

3. Procédé selon la revendication 1, dans lequel le composé d'acide arylboronique est l'acide 3-acétamidophénylboronique.

4. Procédé selon l'une quelconque des revendications 1 à 3, dans lequel le composé immunologiquement actif est la myoglobine.

5. Procédé selon l'une quelconque des revendications 1 à 3, dans lequel le composé immunologiquement actif est une peroxydase.

6. Procédé selon la revendication 5, dans lequel la peroxydase est la peroxydase du raifort.

7. Procédé selon l'une quelconque des revendications 1 à 6, dans lequel la solution est une solution aqueuse.

8. Procédé selon la revendication 7, dans lequel la quantité de composé d'acide arylboronique ajoutée est de 0,01 à 0,5 %, de préférence, d'environ 0,1 %, en poids par volume.

9. Solution ayant un pH de 7,0 ou inférieur à 7,0, laquelle solution comprend un composé immunologiquement actif possédant une activité peroxydase et conjugué à une molécule active choisie dans le groupe constitué par : un anticorps ou fragment de celui-ci, un antigène, un haptène, une pro-téine, une glycoprotéine, un peptide, une pro-téine mimétique, une molécule d'acide nucléique, un stéroïde, une hormone, ou un dérivé de l'une quelconque des molécules précédentes, ladite solution comprenant, en plus, au moins un composé d'acide arylboronique de formule ArB(OH)₂, Ar étant un phényle ou un naphtyle qui est soit non substitué, soit substitué par un ou plusieurs substituants choisis dans le groupe constitué par un alkyle, un alkyle substitué, un aryle, un aryle substitué, un éther, une amine, une amine substituée, un composé azoïque, un amide, un halogène, un nitro, un thiol, un dérivé de thiol, un aldéhyde, un acide, un sel d'acide, un ester, un sulfonate, un phosphonate, et un triméthylsilyle, dans laquelle, quand le composé d'acide arylboronique est substitué par un aryle substitué, le substituant dans l'aryle substitué n'est pas un hydroxyle.

10. Solution selon la revendication 9, dans laquelle le composé d'acide arylboronique est l'acide 4-bromophénylboronique.

11. Solution selon la revendication 9, dans laquelle le composé d'acide arylboronique est l'acide 3-acétamidophénylboronique.

12. Solution selon l'une quelconque des revendications 9 à 11, dans laquelle le réactif comprend, en outre, un agent de conservation.

13. Solution selon l'une quelconque des revendications 9 à 12, dans laquelle le composé immunologiquement actif est la myoglobine.

14. Solution selon l'une quelconque des revendications 9 à 12, dans laquelle le composé immunologiquement actif est une peroxydase.

15. Solution selon la revendication 14, dans laquelle la peroxydase est la peroxydase du raifort.

16. Solution selon l'une quelconque des revendications 9 à 15, dans laquelle la solution est une solution aqueuse.

17. Solution selon la revendication 16, dans laquelle le composé d'acide arylboronique est présent en une quantité qui est de 0,01 à 0,5 %, de préférence, d'environ 0,1 %, en poids par volume.
